# EUROPEAN PATENT SPECIFICATION

(11) **EP 4 218 697 B1**
(45) Date of publication and mention of the grant of the patent: **13.05.2026**
(21) Application number: 23169223.7
(22) Date of filing: 30.08.2018
(51) Int. Cl.: A61F 11/12, B29C 65/02, B29C 65/20, B29C 65/58, B29C 65/56, B29C 65/74, B29C 65/00, A61F 11/08, B29L 31/48, B29L 31/00

(54) **EARPLUG ASSEMBLY AND METHOD FOR FORMING THE SAME**
OHRSTÖPSELANORDNUNG UND VERFAHREN ZUR FORMUNG DAVON
ENSEMBLE BOUCHON D'OREILLE ET SON PROCÉDÉ DE FORMATION

(30) Priority: 01.09.2017 US 201762553620 P
(43) Date of publication of application: 02.08.2023
(62) Divisional of application: 18852128.0
(73) Proprietor: 3M Innovative Properties Company, St. Paul, MN 55133-3427 (US)
(72) Inventor: Ely, Jacob, Saint Paul, MN 54144-1000 (US); Teeters, Kenneth F., Saint Paul, MN 54144-1000 (US); Coffin, Robert C., Saint Paul, MN 54144-1000 (US); Cai, Feng, Saint Paul, MN 54144-1000 (US)
(74) Representative: Mathys & Squire

(56) References cited:
- WO-A1-98/06362
- US-A- 4 293 355
- US-A- 4 867 149
- US-A- 5 668 354
- US-A1- 2005 039 761

## Description

The present disclosure relates to earplug assemblies, e.g., push-to-fit earplugs, where two earplugs are connected by a cord, and methods of manufacturing the earplug assemblies.

The use of hearing protective and noise attenuating devices is well known, and various types of devices have been considered. Such devices include earplugs and semi-aural devices partially or completely constructed of foam or rubber materials that are inserted into, or placed over, the ear canal of a user to physically obstruct the passage of sound waves into the inner ear.

WO 98/06362 presents an acoustical hearing device comprising a foam, and at least one porous component which is mechanically bonded to the foam, where the mechanical bonding of the porous component arises during manufacture wherein the porous component is placed into a mold cavity as an insert, and a foam is formed within the mold so as to allow controlled penetration of the porous component during foaming and importantly, entrapped air in the closed cavity may pass through the porous component during manufacture, thereby allowing the porous component to act as a mold vent.

Compressible or "roll-down" type earplugs generally comprise a compressible, resilient body portion and may be made of suitable slow recovery foam materials. The earplug may be inserted into the ear canal of a user by first rolling it between fingers to compress the body portion, then pushing the body portion into the ear canal, and subsequently allowing the body portion to expand to fill the ear canal.

Push-to-fit type earplugs have also been considered, and may include a compressible attenuating portion and a stiff portion (e.g., a stem) that extends from the attenuating portion. To insert a push-to-fit type earplug, the user grasps the stiff portion and pushes the attenuating portion into the ear canal with an appropriate level of force. The attenuating portion compresses as it is accommodated in the ear canal. Push-to-fit earplugs may allow the earplug to be quickly and easily inserted in an ear canal, and may promote hygiene by minimizing contact with the attenuating portion of the earplug prior to insertion.

A pair of earplugs may be connected by a cord to form an earplug assembly. Although methods to attach the cord to the earplugs are known, they may be costly and may pose manufacturing challenges.

US 4,293, 355 discloses a tethered earplug construction comprising a pair of plasticized thermoplastic foam earplugs tethered together by one or more flexible plasticized thermoplastic cord elements where attachment of the cord element to the earplug is achieved by ultrasonic welding.

### SUMMARY

The invention is defined by the independent claims. The present disclosure relates to earplug assemblies where two earplugs, e.g., push-to-fit earplugs, are connected by a cord, and methods of manufacturing earplug assemblies.

In one aspect a method of providing an earplug assembly according to claim 1 is provided. The earplug assembly comprises a first earplug, a second earplug, and a cord comprising a first end, a second end, and a length extending between the first and second ends, each earplug comprising a stem having a proximal end, a distal end, and a major outer surface and a longitudinal axis extending from the proximal end to the distal end; and a sound attenuating body disposed at the distal end of the stem, wherein the stem comprises a first thermoplastic having a first melting temperature and the cord comprising a second thermoplastic having a second melting temperature that that is within 10°C of or lower than the first melting temperature. The method for preparing the earplug assembly comprises attaching the first and second ends of the cord to the first and second earplugs, respectively, by: inserting a hot tip to a depth into the proximal end of the stem of the earplug to form a cord cavity, the hot tip having a temperature above the first and second melting temperatures; removing the hot tip from the stem; and inserting the end of the cord into the cord cavity to thermally bond the end of the cord to the proximal end of the stem of the earplug.

In another aspect, an earplug assembly according to claim 11 is provided. The earplug assembly comprises a first earplug and a second earplug, each earplug comprising: a stem comprising a proximal end, a distal end, and a longitudinal axis extending from the proximal end to the distal end, the stem comprising a first thermoplastic having a first melting temperature; and a sound attenuating body disposed at the distal end of the stem. The earplug assembly further comprises a cord comprising a first end, a second end, and a length extending between the first and second ends, the cord comprising a second thermoplastic having a second melting temperature, the second melting temperature being within 10°C of or lower than the first melting temperature. The first and second ends of the cord being are inserted into a cord cavity in the proximal ends of the stems of the first and second earplugs, respectively, and thermally bonded to and extending to a depth into the proximal ends of the stems of the first and second earplugs by thermal bonding, the cord cavity of the first and second earplugs being formable by inserting a hot tip into the proximal end of the stem of the respective first and second earplug, the hot tip having a temperature above the first and second melting temperatures.

The above summary is not intended to describe each embodiment or every implementation of the earplug assemblies and methods of manufacturing earplug assemblies as described herein. Rather, a more complete understanding of the invention will become apparent and appreciated by reference to the following Detailed Description and claims in view of the accompanying figures.

### BRIEF DESCRIPTION OF DRAWINGS

FIGURE 1 shows an earplug assembly according to an embodiment.
FIGURE 2A is a partial view of the earplug assembly of FIGURE 1.
FIGURE 2B is a partial cross-sectional view of the earplug assembly of FIGURE 1.
FIGURES 3A-3D show a cartoon depiction of a method of making earplug assembly of FIGURE 1 according to an embodiment.

### DETAILED DESCRIPTION

The present disclosure relates to earplug assemblies including two earplugs connected by a cord. The present disclosure further relates to methods of making earplug assemblies with two earplugs and a cord. The two earplugs of the earplug assembly are connected to the cord by thermal bonding. Favorably, the connection between the earplugs and the cord, or the earplug assembly as a whole, is free of adhesives.

The term "thermally bonded" means a state in which molecules of two materials or surfaces have diffused into the material or surface of the other when in a molten phase such that a bond is formed. Chemical bonding is absent or does not provide the primary source of bonding between thermally bonded materials or surfaces.

The term "thermoplastic" means a polymer that can be repeatedly heated and re-shaped and will retain its shape upon cooling.

The term "thermoset" is used to refer to polymers that are permanently and irreversibly cured and cannot remolded or reheated after their initial curing.

The term "melting temperature" is used to describe a temperature or temperature range at which a thermoplastic polymer transitions from a crystalline or semi-crystalline phase to an amorphous phase. Melting temperature can be determined using various test methods, such as differential scanning calorimetry (DSC).

As used here, the term "comprises" and variations thereof do not have a limiting meaning where these terms appear in the description and claims. Such terms will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they materially affect the activity or action of the listed elements.

In this application, terms such as "a," "an," and "the" are not intended to refer to only a singular entity, but include the general class of which a specific example may be used for illustration. The terms "a," "an," and "the" are used interchangeably with the term "at least one." The phrases "at least one of" and "comprises at least one of" followed by a list refers to any one of the items in the list and any combination of two or more items in the list.

As used here, the term "or" is generally employed in its usual sense including "and/or" unless the content clearly dictates otherwise. The term "and/or" means one or all of the listed elements or a combination of any two or more of the listed elements.

As used here, all numbers are assumed to be modified by the term "about" and in certain situations, preferably, by the term "exactly." As used herein in connection with a measured quantity, the term "about" refers to that variation in the measured quantity as would be expected by the skilled artisan making the measurement and exercising a level of care commensurate with the objective of the measurement and the precision of the measuring equipment used. Herein, "up to" a number (e.g., up to 50) includes the number (e.g., 50).

As used here, the recitations of numerical ranges by endpoints include all numbers subsumed within that range as well as the endpoints (e.g., 1 to 5 includes 1, 1.5, 2, 2.75, 3, 3.80, 4, 5, etc.).

Relative terms such as proximal, distal, left, right, forward, rearward, top, bottom, side, upper, lower, horizontal, vertical, and the like may be used in this disclosure to simplify the description, however, and not to limit the scope of the invention in any way. Terms such as left, right, forward, rearward, top, bottom, side, upper, lower, horizontal, vertical, and the like are from the perspective observed in the particular figure.

Earplug assemblies that provide hearing protection for a user and methods of making earplug assemblies with two earplugs and a connecting cord are described herein. Various types of earplugs can be used as part of the earplug assembly. Although a certain type of earplug is shown in the Figures, this type should not be considered limiting, and other types and shapes of earplugs can be used.

The earplug assembly includes two earplugs connected by a cord. In methods of making earplug assemblies described herein the cord is attached to the earplugs by using a hot tip to create an attachment point (i.e., a cord cavity) at the proximal end of the stem and connecting the end of the cord with the attachment point (e.g., by inserting the end of the cord into the cord cavity) to thermally bond the cord to the earplug. Favorably, the attachment is free of adhesives.

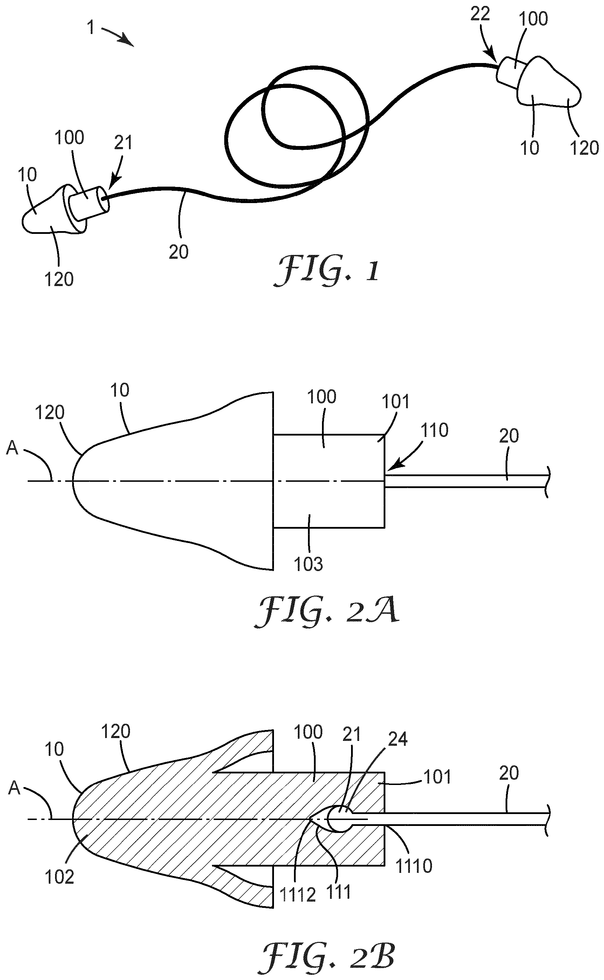

Referring now to FIGURE 1, an exemplary earplug assembly 1 is shown. The earplug assembly 1 includes two earplugs 10 connected by a cord 20..

Various types of earplugs can be used as part of the earplug assembly 1. For example, the earplugs may include a stem or central core, and may additionally include a sound-attenuating body. Alternatively, the earplug stem itself may function as the sound-attenuating body. The earplug assembly 1 shown in the FIGURES includes earplugs 10 with a stem 100 and a sound-attenuating body 120. The stem 100 has a proximal end 101, a distal end 102, and a major outer surface 103, and a longitudinal axis A extending from the proximal end 101 to the distal end 102. The proximal end 101 of the stem 100 is intended to be grasped by the user when inserting or removing the earplug 10 to and from the ear canal.

In the exemplary earplug assembly shown, a sound attenuating body 120 is attached to the major outer surface 103 at the distal end 102 of the stem 100. Generally, the sound-attenuating body 120 may be made of the same material as the stem 100, or a different material. The sound-attenuating body 120 may be made of a compressible material (e.g., a foam or an elastomer) that is suitable for inserting into the ear canal of the user.

The cord 20 has a first end 21 and a second end 22, and a length extending between the first and second ends 21, 22. The length of the cord is not limiting, and any useful cord length can be used. Typical cord lengths vary from about 12 to about 30 inches. The first and second ends 21, 22 are thermally bonded to the stems of the earplugs 10 of the assembly 1. Favorably, the attachment is free of adhesives, or if an adhesive is used, it does not provide the primary mechanism of adhesion between the cord and the earplugs.

FIGURES 2A and 2B show partial views of the exemplary earplug assembly, with FIGURE 2B showing a cross-sectional view of the earplug 10 and one end of the cord 20. The cord 20 is attached to the earplug 10 at an attachment point 110 at the proximal end 101 of the stem 100. As illustrated, the attachment point 110 includes a cord cavity 111, and the end (e.g., first end 21 or second end 22) of the cord 20 extends into the cord cavity 111 and is thermally bonded to the material of the stem 100. A small air gap or cavity may remain at the bottom 1112 of the cord cavity 111 after the cord 20 is attached, as seen in FIGURE 2B.

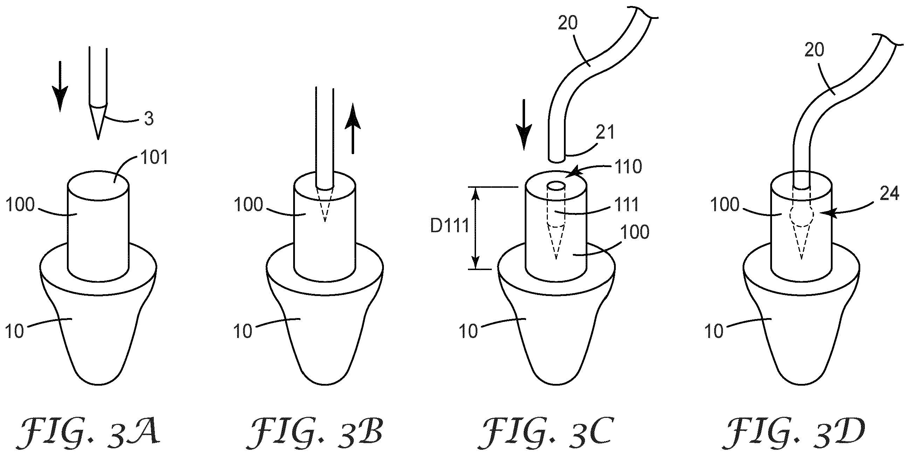

The primary mechanism of retaining the earplug at the end of the cord is thermal bonding. However, in some embodiments, the end of the cord 20 and the cord cavity 111 may additionally form a ball-and-socket attachment, where the end of the cord 20 forms a ball 24 that fits into the cavity 111 (the "socket"). The term "ball" is used here to indicate an enlarged end of the cord. Although a round ball is shown in FIGURES 2B and 3D, the ball 24 can have an irregular shape. The mouth 1110 of the cavity 111 may be narrower than the width of the ball 24, providing a friction fit in addition to the primary adhesion provided by thermal bonding.

The earplugs 10 and the cord 20 may be made of any suitable material. The earplug stem 100 is made from a first material and the cord 20 is made from a second material. As described herein the second material is different from the first material. For example, the first material may be selected to provide suitable characteristics for the stem, which may act as a handle for the earplug and as support for the sound-attenuating portion, or may act as the sound-attenuating portion. The second material may be selected to provide suitable characteristics for the cord, such as flexibility, comfort, durability, etc. Other aspects of the first and second materials may also be considered, such as compatibility with each other in thermal bonding, ease of processing, cost, and environmental considerations.

The stem 100 is made from a first material that includes suitable thermoplastic materials. For example, the first material may include polyolefins, such as polypropylene (PP) or polyethylene (PE), polyethylene terephthalate (PET), polybutylene terephthalate (PBT), poly(methyl methacrylate) (PMMA), acrylonitrile butadiene styrene (ABS), polyamide (nylon), polylactic acid (PLA), polycarbonate, polyether sulfone (PES), polyoxymethylene (POM), polyether ether ketone (PEEK), polyphenylene oxide (PPO), polyphenylene sulfide (PPS), polystyrene (PS), polyvinyl chloride (PVC), and mixtures and alloys thereof. In certain embodiments, the first material is polypropylene, polyethylene terephthalate, polybutylene terephthalate, nylon, polycarbonate, or a mixture or alloy thereof. The first material may be free of PVC.

The first material may be a rigid thermoplastic having a flexural modulus of about 0.3 to about 12 GPa, about 0.5 to about 10 GPa, or about 0.7 to about 8 GPa. In some cases, the first material forms a core that may be further surrounded by a foam material that also forms the sound-attenuating portion 120.

The cord 20 is made from a second material that includes suitable thermoplastic materials, such as thermoplastic polyolefins (TPOs) and thermoplastic elastomers (TPEs). For example, the second material may include ethylene vinyl acetate (EVA), flexible polyvinyl chloride (PVC), Styrene block copolymer (SBC), styrene-butadiene (SB) copolymer, polyurethane, polypropylene-based elastomers, and mixtures and alloys thereof.

The second material may be selected based on its hardness. For example, the second material may be selected from flexible thermoplastics that have type A shore hardness of about 25 to about 90, or about 30 to about 75, or about 35 to about 60. In certain embodiments the second material includes or is ethylene vinyl acetate (EVA). The second material may be free of PVC.

The second thermoplastic material is selected to have a melting temperature (e.g., the second melting temperature) that is within about 10 °C of or lower than the melting temperature of the first thermoplastic material (e.g., the first melting temperature). For example, the second material may have a melting temperature that is about 0 to about 200 °C lower, about 0 to about 150 °C lower, about 0 to about 100 °C lower, about 0 to about 75 °C lower, about 0 to about 50 °C lower, or about 0 to about 35 °C lower than the melting temperature of the first material. In one example, the first material has a melting temperature of about 150 to about 200 °C, and the second material has a melting temperature of about 70 to about 80 °C. When melting temperatures of the two materials are compared, the same method (e.g., DSC) should be used to determine each melting temperature.

In methods of making earplug assemblies described here, the cord is coupled with the stems of two earplugs to connect the earplugs in an earplug assembly that includes a first earplug, a second earplug, and a cord connecting the first and second earplugs, the cord having a first end, a second end, and a length extending between the first and second ends. The methods of making earplug assemblies described herein may reduce the difficulty and/or the cost of manufacturing earplug assemblies as described herein. For example, the methods may make manufacturing faster, simpler, and cheaper.

A simplified schematic of an exemplary method is shown in FIGURES 3A-3D. A hot tip 3 is contacted to the proximal end 101 of the stem 100 to melt a portion of the material of the stem 100 to create an attachment point 110. The hot tip 3 may contact the stem 100 approximately at the center of the proximal end 101. The hot tip 3 is inserted into the stem 100 to a depth to create a cord cavity 111.

The hot tip 3 has a temperature that is above the first and second melting temperatures of the first and second thermoplastic materials of the stem and cord. For example, the hot tip 3 may have a temperature of about 150 to about 450 °C, about 200 to about 425 °C, about 225 to about 400 °C, or about 250 to about 350 °C. The temperature of the hot tip 3 can be selected such that it is at least about 30 °C, at least 40 °C, at least 50 °C, at least 75 °C, or at least 100 °C above the melting temperature of the first material.

The hot tip 3 is removed from the stem 100 after a short period of time. The timing depends in part on the difference between the temperature of the hot tip 3 and the melting temperature of the first material. The greater the difference, the sooner the hot tip 3 can be removed. For example, when the difference is about 75 °C or greater, the hot tip 3 may be removed after about 0.1 to about 5 seconds, or about 0.2 to about 2 seconds. A greater length of time may be needed if the difference is smaller.

When the hot tip 3 contacts the proximal end 101 of the stem 100, it raises the temperature and melts a portion of the material of the stem 100. The end of the cord 20 can then be contacted with the melted portion at the attachment point 110. In other words, as illustrated since the hot tip 3 was inserted into the stem 100 to create a cord cavity 111 with a depth D111, the end of the cord (e.g., first end 21 or second end 22) can be then inserted into the cord cavity 111. The depth D111 of the cord cavity may be about 2 to about 10 mm, or about 3 to about 7 mm.

Typically, at the time when the cord 20 is contacted with the attachment point 110, the temperature of the melted portion of the stem 100 is higher than the melting temperature of the cord material (second material). The temperature of the melted portion is favorably sufficient to melt a portion of the end of the cord 20, thus thermally bonding the cord 20 to the earplug 10. In at least some cases, the heat source (e.g., the hot tip 3) is not applied to the cord, but only the stem of the earplug. Moreover, the end of the cord 20 is melted by the residual heat of the stem 100. The end of the cord 20 is favorably contacted with the attachment point 110 soon after the hot tip 3 is removed and before the attachment point has cooled to a temperature below the melting temperature of the second material (the material of the cord 20).

The end of the cord 20 may be inserted all the way to the bottom of the cord cavity 111 (the insertion depth is equal to the depth D111 of the cord cavity 111). However, in some cases, the cord 20 is inserted only part of the way into the cord cavity 111 (the insertion depth is less than the depth D111 of the cord cavity 111). For example, the end of the cord 20 may inserted into the cord cavity 111 so that a small air gap or cavity remains at the bottom 1112 of the cord cavity 111 after the cord 20 is attached, as seen in FIGURE 2B.

The end of the cord 20 is favorably held in place at the proximal end 101 of the stem 100 (i.e. in the cord cavity 111) for a length of time to allow for the thermal bonding to occur. For example, the cord 20 can be held in place for about 0.5 to about 4 seconds. After thermal bonding has occurred and the temperature of the material at the attachment point has cooled sufficiently (e.g., below the melting temperature of at least one of the materials), the cord will stay attached to the earplug without assistance (e.g., without holding).

In some embodiments, the melting of a portion of the end of the cord 20 may form a ball-and-socket type attachment of the cord in the cord cavity 111. As seen in FIGURES 2B and 3D, the end of the cord 20 may form an enlarged ball 24 inside the cord cavity 111, providing a friction fit in addition to the primary adhesion method of thermal bonding. The surrounding core material may at least partially conform to the shape of the ball 24.

In an exemplary embodiment of the method for preparing an earplug assembly as described herein, the melting temperature of the second thermoplastic material may be lower than the melting temperature of the first thermoplastic material by 20°C or more.

### EXAMPLE

A cord was attached to an earplug using the method described above. The earplug had a polypropylene (PP) composite core extending through the stem, surrounded by a layer of material that was foamed at the distal end to form a sound-attenuating portion. The polypropylene core was exposed at the proximal end of the stem. The cord was 1.27 mm thick ethylene vinyl acetate (EVA) with a melting temperature of approximately 74 °C. The melting temperature of the PP core was in the range of 165-200 °C.

A metal tip with a thickness of about 1.57 mm and a pointed end was heated to a temperature of about 370 °C and inserted into the proximal end of the PP core of the stem to create a cord cavity. The metal tip was held in the cord cavity for about 1 second, causing a portion of the core to melt. After the metal tip was removed from the cord cavity, the end of the cord was inserted into the cord cavity and was held in place for about 3 seconds.

It was observed that the end of the cord was thermally bonded to the stem of the earplug. It was further observed that a portion of the cord had melted and formed a ball inside the cord cavity, forming a ball-and-socket type connection. The connection between the cord and the earplug was found to be sufficiently strong for use in earplug assemblies without the use of adhesives.

Various modifications and alterations to this disclosure will become apparent to those skilled in the art without departing from the scope of the present invention. It should be understood that this disclosure is not intended to be unduly limited by the illustrative embodiments and examples set forth herein and that such examples and embodiments are presented by way of example only with the scope of the invention limited only by the claims set forth here.

## Claims

1. A method for preparing an earplug assembly, the earplug assembly (1) comprising:
a first earplug, a second earplug, ,
each earplug (10) comprising:
a stem (100) having a proximal end (101), a distal end (102), and a longitudinal axis (A) extending from the proximal end to the distal end, the stem comprising a first thermoplastic having a first melting temperature; and
a sound attenuating body (120) disposed at the distal end of the stem; and
a cord (20) comprising a first end (21), a second end (22), and a length extending between the first and second ends, the cord comprising a second thermoplastic having a second melting temperature that is within 10°C of or lower than the first melting temperature;
the method comprising attaching the first and second ends of the cord to the first and second earplugs, respectively, by:
inserting a hot tip (3) to a depth (D111) into the proximal end of the stem of the respective first or second earplug to form a cord cavity (111), the hot tip having a temperature above the first and second melting temperatures;
removing the hot tip from the stem; and
inserting the respective first or second end of the cord into the cord cavity to thermally bond the first or second end of the cord to the proximal end of the stem of the first or second earplug, respectively.

2. The method of claim 1, wherein the inserting the first or second end (21, 22) of the cord (20) into the cord cavity (111) melts a portion of the end of the cord.

3. The method of claim 1 or 2, wherein the method forms a ball-and-socket attachment of the end (21, 22) of the cord (20) in the cord cavity (111).

4. The method of any one of claims 1 to 3, further comprising holding the end (21, 22) of the cord (20) in the cord cavity (111) for about 0.5 to about 4 seconds.

5. The method of any one of claims 1 to 4, wherein the melting temperature of the second thermoplastic is lower than the melting temperature of the first thermoplastic by 20 °C or more.

6. The method of any one of claims 1 to 5, wherein the first thermoplastic is selected from rigid thermoplastics having a flexural modulus of about 0.3 to about 12 GPa and/or wherein the second thermoplastic is selected from flexible thermoplastics having a type A shore hardness of about 25 to about 90.

7. The method of any one of claims 1 to 6, wherein the first thermoplastic comprises polypropylene, polyethylene terephthalate, polybutylene terephthalate, nylon, polycarbonate, or a mixture thereof and/or the second thermoplastic comprises thermoplastic polyolefins, thermoplastic elastomers, ethylene vinyl acetate, flexible polyvinyl chloride, styrene-butadiene copolymer, polypropylene-based elastomers, or a mixture thereof.

8. The method of any one of claims 1 to 7, wherein the hot tip (3) has a temperature of about 175 to about 425 °C.

9. The method of any one of claims 1 to 8, wherein the hot tip (3) is removed from the stem (100) about 0.1 to about 2 seconds after inserting the hot tip.

10. The method of any one of claims 1 to 9, wherein the hot tip (3) is inserted to a depth of about 2 to about 10 mm into the proximal end (101) of the stem (100).

11. An earplug assembly (1) comprising:
a first earplug and a second earplug,
each earplug (10) comprising:
a stem (100) comprising a proximal end (101), a distal end (102), and a longitudinal axis (A) extending from the proximal end to the distal end, the stem comprising a first thermoplastic having a first melting temperature; and
a sound attenuating body (120) disposed at the distal end of the stem; and
a cord (20) comprising a first end (21), a second end (22), and a length extending between the first and second ends, the cord comprising a second thermoplastic having a second melting temperature, the second melting temperature being within 10°C of or lower than the first melting temperature,
the first and second ends of the cord being inserted into a cord cavity (111) in the proximal ends of the stems of the first and second earplugs, respectively, and being thermally bonded to and extending to a depth into the proximal ends of the stems of the first and second earplugs by thermal bonding, and
the cord cavity of the first and second earplugs being formable by inserting a hot tip (3) into the proximal end of the stem of the respective first and second earplug, the hot tip having a temperature above the first and second melting temperatures.

12. The earplug assembly (1) of claim 11, wherein the second melting temperature is lower than the first melting temperature by at least 20 °C.

13. The earplug assembly (1) of claim 12, wherein the attachment of the cord (20) to the stem (100) is free of adhesives.

14. The earplug assembly of claim 12 or 13, wherein the first thermoplastic comprises polypropylene, nylon, or a combination thereof.

15. The earplug assembly of any one of claims 12 to 14, wherein the second thermoplastic is ethylene vinyl acetate, EVA.

## Patentansprüche

1. Ein Verfahren zum Herstellen einer Ohrstöpselanordnung, wobei die Ohrstöpselanordnung (1) aufweist:
einen ersten Ohrstöpsel, einen zweiten Ohrstöpsel,
wobei jeder Ohrstöpsel (10) aufweist:
einen Schaft (100), der ein proximales Ende (101), ein distales Ende (102) und eine Längsachse (A) aufweist, die sich von dem proximalen Ende zu dem distalen Ende erstreckt; wobei der Schaft ein erster Thermoplast mit einer ersten Schmelztemperatur aufweist; und
einen Schalldämpfungskörper (120), der an dem distalen Ende des Schafts angeordnet ist; und
ein Kabel (20), das ein erstes Ende (21), ein zweites Ende (22) und eine sich zwischen dem ersten und dem zweiten Ende erstreckende Länge aufweist, wobei das Kabel einen zweiten Thermoplasten mit einer zweiten Schmelztemperatur aufweist, die innerhalb von 10 °C oder niedriger als die erste Schmelztemperatur liegt;
wobei das Verfahren Befestigen des ersten und zweiten Endes des Kabels jeweils an den ersten und den zweiten Ohrstöpsel aufweist durch:
Einführen einer heißen Spitze (3) zu einer Tiefe (D111) in das proximale Ende des Schafts des jeweiligen ersten oder zweiten Ohrstöpsels, um einen Kabelhohlraum (111) zu bilden, wobei die heiße Spitze eine Temperatur über der ersten und der zweiten Schmelztemperatur aufweist;
Entfernen der heißen Spitze aus dem Schaft; und
Einführen des jeweiligen ersten oder zweiten Endes des Kabels in den Kabelhohlraum, um das erste oder das zweite Ende des Kabels jeweils mit dem proximalen Ende des Schafts des ersten oder des zweiten Ohrstöpsels thermisch zu verbinden.

2. Das Verfahren nach Anspruch 1, wobei das Einführen des ersten oder zweiten Endes (21, 22) des Kabels (20) in den Kabelhohlraum (111) einen Abschnitt des Endes des Kabels schmilzt.

3. Das Verfahren nach Anspruch 1 oder 2, wobei das Verfahren eine Kugelkopfbefestigung des Endes (21, 22) des Kabels (20) in dem Kabelhohlraum (111) bildet.

4. Das Verfahren nach Anspruch 1 bis 3, ferner aufweisend Halten des Endes (21, 22) des Kabels (20) in dem Kabelhohlraum (111) für etwa 0,5 bis etwa 4 Sekunden.

5. Das Verfahren nach Anspruch 1 bis 4, wobei die Schmelztemperatur des zweiten Thermoplasten um 20 °C oder mehr niedriger als die Schmelztemperatur des ersten Thermoplasten ist.

6. Das Verfahren nach einem der Ansprüche 1 bis 5, wobei der erste Thermoplast aus starren Thermoplasten mit einem Biegemodul von etwa 0,3 bis etwa 12 GPa ausgewählt ist und/oder wobei der zweite Thermoplast aus flexiblen Thermoplasten mit einer Shore-A-Härte von etwa 25 bis etwa 90 ausgewählt ist.

7. Das Verfahren nach einem der Ansprüche 1 bis 6, wobei der erste Thermoplast Polypropylen, Polyethylenterephthalat, Polybutylenterephthalat, Nylon, Polycarbonat oder eine Mischung davon aufweist und/oder der zweite Thermoplast thermoplastische Polyolefine, thermoplastische Elastomere, Ethylen-Vinylacetat, flexibles Polyvinylchlorid, Styrol-Butadien-Copolymer, Polypropylen-basierte Elastomere oder eine Mischung davon aufweist.

8. Das Verfahren nach Anspruch 1 bis 7, wobei die heiße Spitze (3) eine Temperatur von etwa 175 bis etwa 425 °C aufweist.

9. Das Verfahren nach Anspruch 1 bis 8, wobei die heiße Spitze (3) etwa 0,1 bis etwa 2 Sekunden nach Einführen der heißen Spitze von dem Schaft (100) entfernt wird.

10. Das Verfahren nach Anspruch 1 bis 9, wobei die heiße Spitze (3) zu einer Tiefe von etwa 2 bis etwa 10 mm in das proximale Ende (101) des Schafts (100) eingeführt wird.

11. Eine Ohrstöpselanordnung (1), aufweisend:
einen ersten Ohrstöpsel und einen zweiten Ohrstöpsel,
wobei jeder Ohrstöpsel (10) aufweist:
einen Schaft (100), der ein proximales Ende (101), ein distales Ende (102) und eine Längsachse (A) aufweist, die sich von dem proximalen Ende zu dem distalen Ende erstreckt; wobei der Schaft einen ersten Thermoplasten mit einer ersten Schmelztemperatur aufweist; und
einen Schalldämpfungskörper (120), der an dem distalen Ende des Schafts angeordnet ist; und
ein Kabel (20), das ein erstes Ende (21), ein zweites Ende (22) und eine sich zwischen dem ersten und dem zweiten Ende erstreckende Länge aufweist, wobei das Kabel einen zweiten Thermoplasten mit einer zweiten Schmelztemperatur aufweist, wobei die zweite Schmelztemperatur innerhalb von 10 °C oder niedriger als die erste Schmelztemperatur ist,
wobei das erste und das zweite Ende des Kabels in einen Kabelhohlraum (111) in die proximalen Enden der Schäfte des ersten bzw. des zweiten Ohrstöpsels eingeführt werden und sich durch thermisches Verbinden bis zu einer Tiefe in die proximalen Enden der Schäfte des ersten und des zweiten Ohrstöpsels erstrecken, und
wobei der Kabelhohlraum des ersten und des zweiten Ohrstöpsels durch Einführen einer heißen Spitze (3) in das proximale Ende des Schafts des jeweiligen ersten und zweiten Ohrstöpsels formbar ist, wobei die heiße Spitze eine Temperatur oberhalb der ersten und der zweiten Schmelztemperatur aufweist.

12. Die Ohrstöpselanordnung (1) nach Anspruch 11, wobei die zweite Schmelztemperatur um mindestens 20 °C niedriger als die erste Schmelztemperatur ist.

13. Die Ohrstöpselanordnung (1) nach Anspruch 12, wobei die Befestigung des Kabels (20) an dem Schaft (100) frei von Kleber ist.

14. Die Ohrstöpselanordnung nach Anspruch 12 oder 13, wobei der erste Thermoplast Polypropylen, Nylon oder eine Kombination davon aufweist.

15. Die Ohrstöpselanordnung nach einem der Ansprüche 12 bis 14, wobei der zweite Thermoplast Ethylenvinylacetat (EVA) ist.

## Revendications

1. Procédé de préparation d'un ensemble bouchon d'oreille, l'ensemble bouchon d'oreille (1) comprenant :
un premier bouchon d'oreille, un second bouchon d'oreille,
chaque bouchon d'oreille (10) comprenant :
une tige (100) ayant une extrémité proximale (101), une extrémité distale (102), et un axe longitudinal (A) s'étendant de l'extrémité proximale à l'extrémité distale, la tige comprenant un premier thermoplastique ayant une première température de fusion ; et
un corps d'atténuation de son (120) disposé au niveau de l'extrémité distale de la tige ; et
un cordon (20) comprenant une première extrémité (21), une seconde extrémité (22), et une longueur s'étendant entre les première et seconde extrémités, le cordon comprenant un second thermoplastique ayant une seconde température de fusion qui est à moins de 10 °C de la première température de fusion ou inférieure à celle-ci ;
le procédé comprenant la fixation des première et seconde extrémités du cordon aux premier et second bouchons d'oreille, respectivement, par :
l'insertion d'une pointe chaude (3) jusqu'à une profondeur (D111) dans l'extrémité proximale de la tige du premier ou second bouchon d'oreille respectif pour former une cavité de cordon (111), la pointe chaude ayant une température supérieure aux première et seconde température de fusion ;
le retrait de la pointe chaude hors de la tige ; et
l'insertion de la première ou seconde extrémité respective du cordon dans la cavité de cordon, pour lier thermiquement la première ou seconde extrémité du cordon à l'extrémité proximale de la tige du premier ou second bouchon d'oreille, respectivement.

2. Procédé selon la revendication 1, dans lequel l'insertion de la première ou seconde extrémité (21, 22) du cordon (20) dans la cavité de cordon (111) fait fondre une partie de l'extrémité du cordon.

3. Procédé selon la revendication 1 ou 2, dans lequel le procédé forme une fixation à rotule de l'extrémité (21, 22) du cordon (20) dans la cavité de cordon (111).

4. Procédé selon l'une quelconque des revendications 1 à 3, comprenant en outre le maintien de l'extrémité (21, 22) du cordon (20) dans la cavité de cordon (111) pendant environ 0,5 à environ 4 secondes.

5. Procédé selon l'une quelconque des revendications 1 à 4, dans lequel la température de fusion du second thermoplastique est inférieure à la température de fusion du premier thermoplastique de 20 °C ou plus.

6. Procédé selon l'une quelconque des revendications 1 à 5, dans lequel le premier thermoplastique est choisi parmi les thermoplastiques rigides ayant un module de flexion d'environ 0,3 à environ 12 GPa et/ou dans lequel le second thermoplastique est choisi parmi les thermoplastiques souples ayant une dureté Shore de type A d'environ 25 à environ 90.

7. Procédé selon l'une quelconque des revendications 1 à 6, dans lequel le premier thermoplastique comprend du polypropylène, du polyéthylène téréphtalate, du polybutylène téréphtalate, du nylon, du polycarbonate, ou un mélange de ceux-ci, et/ou le second thermoplastique comprend des polyoléfines thermoplastiques, des élastomères thermoplastiques, de l'éthylène-acétate de vinyle, du chlorure de polyvinyle souple, un copolymère styrène-butadiène, des élastomères à base de polypropylène, ou un mélange de ceux-ci.

8. Procédé selon l'une quelconque des revendications 1 à 7, dans lequel la pointe chaude (3) a une température d'environ 175 à environ 425 °C.

9. Procédé selon l'une quelconque des revendications 1 à 8, dans lequel la pointe chaude (3) est retirée de la tige (100) environ 0,1 à environ 2 secondes après l'insertion de la pointe chaude.

10. Procédé selon l'une quelconque des revendications 1 à 9, dans lequel la pointe chaude (3) est insérée jusqu'à une profondeur d'environ 2 à environ 10 mm dans l'extrémité proximale (101) de la tige (100).

11. Ensemble bouchon d'oreille (1) comprenant :
un premier bouchon d'oreille et un second bouchon d'oreille,
chaque bouchon d'oreille (10) comprenant :
une tige (100) comprenant une extrémité proximale (101), une extrémité distale (102), et un axe longitudinal (A) s'étendant de l'extrémité proximale à l'extrémité distale, la tige comprenant un premier thermoplastique ayant une première température de fusion ; et
un corps d'atténuation de son (120) disposé au niveau de l'extrémité distale de la tige ; et
un cordon (20) comprenant une première extrémité (21), une seconde extrémité (22), et une longueur s'étendant entre les première et seconde extrémités, le cordon comprenant un second thermoplastique ayant une seconde température de fusion, la seconde température étant à moins de 10 °C de la première température de fusion ou inférieure à celle-ci,
les première et seconde extrémités du cordon étant insérées dans une cavité de cordon (111) dans les extrémités proximales des tiges des premier et second bouchons d'oreille, respectivement, et étant thermiquement liées aux extrémités proximales des tiges des premier et second bouchons d'oreille et s'étendant jusqu'à une profondeur dans celles-ci par liaison thermique, et
la cavité de cordon des premier et second bouchons d'oreille pouvant être formée en insérant une pointe chaude (3) dans l'extrémité proximale de la tige des premier et second bouchons d'oreille respectifs, la pointe chaude ayant une température supérieure aux première et seconde températures de fusion.

12. Ensemble bouchon d'oreille (1) selon la revendication 11, dans lequel la seconde température de fusion est inférieure à la première température de fusion d'au moins 20 °C.

13. Ensemble bouchon d'oreille (1) selon la revendication 12, dans lequel la fixation du cordon (20) à la tige (100) est exempte d'adhésifs.

14. Ensemble bouchon d'oreille selon la revendication 12 ou 13, dans lequel le premier thermoplastique comprend du polypropylène, du nylon, ou une combinaison de ceux-ci.

15. Ensemble bouchon d'oreille selon l'une quelconque des revendications 12 à 14, dans lequel le second thermoplastique est l'éthylène-acétate de vinyle, EVA.
